# EUROPEAN PATENT SPECIFICATION

(11) **EP 0 336 768 B1**
(45) Date of publication and mention of the grant of the patent: **22.12.1993**
(21) Application number: 89303434.8
(22) Date of filing: 07.04.1989
(51) Int. Cl.: B01J 23/00, B01J 23/10, B01J 23/26, B01J 23/34, C07B 33/00, C07B 41/00, C07C 45/36, C07C 45/38, C07C 47/52, C07C 49/786

(54) **Catalyst and its use in oxidation**
Katalysator und dessen Anwendung in Oxydation
Catalyseur et son application dans l'oxydation

(30) Priority: 08.04.1988 GB 8808240
(43) Date of publication of application: 11.10.1989
(73) Proprietor: CONTRACT CHEMICALS (KNOWSLEY) LIMITED, Warrington Cheshire WA1 1DL (GB)
(72) Inventor: Brown, Christopher Martin, Callands Warrington Cheshire (GB); McQuarrie, Duncan James, Winsford Cheshire (GB); Clark, James Hanley, York (GB); Kybett, Adrian Peter, Tadcaster York Yorkshire (GB)
(74) Representative: Gore, Peter Manson

(56) References cited:
- DE-A- 1 267 675
- DE-A- 2 737 511
- FR-A- 1 231 065
- FR-A- 2 252 126
- FR-A- 2 263 818
- US-A- 2 169 368
- US-A- 4 289 919

## Description

The present invention relates to a catalyst and to the use of a catalyst in oxidation.

Many processes are known for the oxidation of organic compounds. However, there are also many disadvantages associated with such known processes. It has not been possible to provide a satisfactory process which is operable at moderate temperatures and pressures and is sufficiently rapid when using molecular oxygen in relatively dilute form, such as, for example, air. There have also been problems associated with the use of chemicals which may be dangerous and/or lead to environmental problems arising, for example, in treatment of a catalyst to enable it to be re-used. There may be considered to be at least four groups of oxidation processes using known oxidants to effect the oxidation reaction.

One such oxidation process is the Cr^{VI} or similar type of oxidation. Such a process is referred to, for example, in US-A- 2 794 813. A major problem with such an oxidation is that the Cr^{VI} oxidant does not have a catalytic nature. Also regeneration of the oxidant after separation does not appear possible so that large amounts of toxic effluent are generated. A similar type of oxidation involving the use of Ce^{IV} oxidant is disclosed in EP-A- 0205173. In that reaction the Ce^{IV} oxidant is non-catalytic although it can be regenerated electrochemically after the oxidation. Another oxidation process utilizes nitric acid as the oxidant (see, for example, SE-137686). However, since the nitric acid is usually used in concentrated form, it is a very corrosive liquid and also produces large amounts of corrosive gas. Additionally, problems can be encountered due to side reactions such as, for example, nitration which can lead to potentially dangerous products. Attempts have been made to overcome this particular problem by the use of diluted nitric acid, but such a solution in turn leads to what may be described as "poor vessel occupancy". Often it is necessary to use an excess of nitric acid. Pressure and relatively high temperatures, for example, 150°C to 200°C, are also often necessary.

A further oxidation process involves the use of Co^{II} salts and the like in acetic acid or similar solution. Such a type of oxidation is described, for example, in DE- 1 267 675 and DD- 125 753. Whilst such a type of process is catalytic in nature it does require not only the use of an acidic solvent but also high temperatures and pressure. Such conditions can lead to explosive mixtures being generated so that careful monitoring of the reaction parameters is necessary.

A still further oxidation proces is a base catalysed reaction. Such a process is referred to, for example, in US- A- 286 766 and US- A- 3 647 886, the latter mentioning the use of potassium fluoride which does function as a base under appropriate conditions (see, for example, J.H. Clark, Chem. Rev. 80, 1980, p420). However, such a type of process usually requires the use of high pressure even when oxygen itself is employed.

It has now been found possible to provide a novel catalyst whose use in oxidation substantially overcomes the problems referred to above and particularly may be used in an oxidation reaction in which the oxygen is in the form of air.

According to the present invention there is provided an oxidation catalyst which comprises a dichromate ion chemisorbed on alumina or a permanganate ion chemisorbed on alumina.

It will be appreciated that alumina has not been regarded as a catalyst. The alumina used in the present invention is usually one having a high surface area, preferably 100 to 300 m²/g.

One method of preparing an oxidation catalyst comprises firstly preparing a solution of a reagent containing a permanganate or dichlorate compound in a suitable solvent. The solvent may be, for example, water, although other solvents may be used. Alumina is preferably added to the solution so that the amount of alumina present is usually in the range of from 1 gram of alumina per 0.01 m mol of the reagent up to 1 gram of alumina per 4 m mol of the reagent. The mixture is usually stirred. The pH of the solution may be adjusted, either before or after the addition of the alumina, by the additon of acid or base. The pH should preferably be 7 or less. The solvent can be then removed under vacuum to produce a finely divided solid.

Another method of preparing an oxidation catalyst comprises mixing a solution of 0.1 to 1.0 mol dm⁻³ permanganate or dichromate ion in water with alumina. If 250 ml of the solution is used then the amount of alumina added should preferably be up to 50g.

The supported catalyst may be used as so prepared or is preferably further treated prior to use as an oxidation catalyst. Such further treatment may be, for example, washing and/or thermal drying. In any of the examples of catalyst preparation given it is intended to achieve a dispersion of the permanganate or dichromate ion on the alumina surface. This dispersion may be of a physisorbed, or physisorbed and chemisorbed or, preferably of a chemisorbed nature. Chemisorption is distinguished here from physisorption in that in the former case the permanganate or dichromate species may not be removed from the alumina surface by water.

According to another embodiment of the present invention there is provided a process for the oxidation of an organic compound which comprises reacting an organic substrate with a gas containing free oxygen in the presence of the catalyst of the present invention on which comprises a catalyst prepared according to the method of the present invention.

Suitable organic substrates which may be used in the process of the present invention include those compounds having the general formulae:
(a) R¹R²R³ CH
(b) 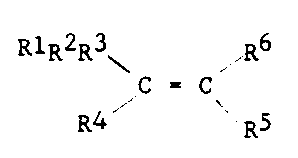
(c) R - C ≡ C - CR¹R²R³
(d) R¹R²R³C - COR⁴
(e) R¹R²R³C OCOR⁴
(f) R¹R²R³C NR⁴R⁵
(g) R¹R²R³C CO₂R⁴
(h) R¹R²R³C CX¹X²X³
(i) R¹R²CHOH
(j) H(orD)R¹CO
(k) R¹R²C = CR³R⁴
(l) R¹R²R³CX
wherein R and R¹ to R⁶ may independently each be H, D (deuterium), halogen, alkyl, aryl, NO₂ or CN; and
X, X¹ X² and X³ may independently each be fluorine, chlorine, bromine or iodine.

The various R groups may be substituted by any conbination of the conventional substituents. Substituents may be, or may contain rings, or combinations of substituents may form rings.

Other organic substrates which may be used in the process of the present invention include:
(1) Condensed aromatic compounds such as, for example, napthalene, anthracene or phenanthene, as well as phenols;
(2) Sulphur containing compounds; and
(3) Heterocyclic compounds.

The substrate to be oxidized may, for example, be mixed with an appropriate amount of the catalyst of the present invention, and a stream of air blown through the stirred mixture which is maintained at a desired temperature.

Alternatively air under a suitable pressure may be used in a closed system.

If desired a solvent may also be employed.

The reaction is continued for an appropriate time and the catalyst is then separated by filtration, or a similar technique. In contrast with many known processes, the catalyst may be re-used.

The oxidation product may be purified by any conventional means.

The present invention will now be further described with reference to, but is in no manner limited to, the following Examples.

### Example 1

Oxidation of diphenylmethane. A catalyst was prepared by dissolving 45m mol of potassium dichromate in 250 ml water. 15g of alumina were added, and the mixture stirred for 2 hours. The catalyst was then obtained by filtering the mixture and washing the solid with water until no further dichromate was removed. The solid was then dried such that a free flowing powder is obtained. The amount of dichromate ion remaining on the alumina was 0.075m mol per gram of alumina.

6g of the catalyst so prepared were added to 100g of diphenylmethane at 150°C with an air flow into the mixture of 800 ml per minute, and with efficient mechanical stirring. Thus between 1 and 2% of the diphenylmethane were oxidised per hour. For example, after 48 hours 64% of the diphenylmethane were converted to benzophenone. This represented 84400% with respect to the Cr.

### Example 2

### Oxidation of benzyl alcohol

The catalyst prepared as described in Example 1 was used, with the same quantities and conditions, to oxidize benzyl alcohol. It was oxidised at between 1 and 2% per hour. After 16 hours, 16% of the benzyl alcohol was converted to benzaldehyde. This represented 32900% with respect to the Cr.

### Example 3

### Oxidation of chlorodiphenylmethane.

The catalyst prepared as described in Example 1 was used, with the same quantities and conditions, to oxidize chlorodiphenylmethane. It was oxidised at between 4% and 5% per hour.

### Example 4

### Oxidation of diphenylmethane

A catalyst was prepared with potassium permanganate in place of dichromate in the manner described in Example 1. The catalyst so obtained oxidised diphenylmethane at between 1% and 2% per hour. After 61 hours 70% of the diphenylmethane was converted to benzophenone. This represented 347000% with respect to the permanganate.

## Claims

1. An oxidation catalyst which comprises a dichromate ion chemisorbed on alumina or a permangante ion chemisorbed on alumina.

2. A method for the preparation of an oxidation catalyst as claimed in claim 1 which comprises preparing a solution of a reagent containing a permanganate or dichromate compound in a solvent, adding alumina to the solution and mixing, followed by removal of the solvent.

3. A method according to claim 2, wherein the supported catalyst so obtained is further treated by washing and/or thermal drying.

4. A method according to claim 2 or 3, wherein the mixture of alumina and solution containing the reagent of permanganate or dichromate compound contains from 1 gram of alumina per 0.01 m mol of the reagent up to 1 gram of alumina per 4 m mol of the reagent.

5. A method according to any of claims 2 to 4, wherein the pH of the solution is adjusted, either before or after the addition of alumina, to 7 or less.

6. A method for the oxidation of an organic compound which comprises reacting an organic substrate with a gas containing free oxygen in the presence of a catalyst as claimed in claim 1 or as prepared as claimed in any of claims 2 to 5.

## Patentansprüche

1. Oxidationskatalysator, der auf Tonerde chemisch sorbierte Dichromat- oder Permanganationen umfaßt.

2. Verfahren zur Herstellung eines Oxidationskatalysators nach Anspruch 1, das das Lösen eines eine Permanganat- oder Dichromatverbindung enthaltenden Reagens in einem Lösungsmittel, Zugabe von Tonerde zur Lösung, Mischen und nachfolgende Entfernung des Lösungsmittels umfaßt.

3. Verfahren nach Anspruch 2, worin der so erhaltene Trägerkatalysator durch Waschen und/oder Trocknung unter Wärmeeinwirkung weiterbehandelt wird.

4. Verfahren nach einem der Ansprüche 2 oder 3, worin das Gemisch aus Tonerde und der das Reagens Permanganat- oder Dichromatverbindung enthaltenden Lösung, 1 g Tonerde pro 0.01 mM des Reagens bis 1 g Tonerde pro 4 mM des Reagens enthält.

5. Verfahren nach einem der Ansprüche 2 bis 4, worin der pH der Lösung entweder vor oder nach der Zugabe der Tonerde auf 7 oder darunter eingestellt wird.

6. Verfahren zur Oxidation einer organischen Verbindung, welches die Umsetzung eines organischen Substrats mit einem freien Sauerstoff enthaltenden Gas in Anwesenheit eines nach Anspruch 1 beanspruchten bzw. gemäß einem der Ansprüche 2 bis 5 hergestellten Katalysators umfaßt.

## Revendications

1. Catalyseur d'oxydation caractérisé en ce qu'il comprend un ion dichromate adsorbé chimiquement sur de l'alumine, ou un ion permanganate adsorbé chimiquement sur de l'alumine.

2. Procédé pour la préparation d'un catalyseur d'oxydation selon la revendication 1, procédé caractérisé en ce qu'il consiste à préparer une solution d'un réactif contenant un composé de permanganate ou de dichromate dans un solvant, à ajouter de l'alumine à la solution et à mélanger, puis à retirer le solvant.

3. Procédé selon la revendication 2, caractérisé en ce que le catalyseur supporté ainsi obtenu est en outre traité par lavage et/ou séchage thermique.

4. Procédé selon l'une quelconque des revendications 2 et 3, caractérisé en ce que le mélange d'alumine et de la solution contenant le réactif de composé de permanganate ou de dichromate, contient de 1 gramme d'alumine pour 0,01 mmol du réactif, jusqu'à 1 gramme d'alumine pour 4 mmol du réactif.

5. Procédé selon l'une quelconque des revendications 2 à 4, caractérisé en ce que le pH de la solution est réglé à 7 ou moins soit avant, soit après l'addition d'alumine.

6. Procédé pour l'oxydation d'un composé organique consistant à faire réagir une substance organique avec un gaz contenant de l'oxygène libre en présence d'un catalyseur selon la revendication 1 ou préparé selon l'une quelconque des revendications 2 à 5.
